# EUROPEAN PATENT APPLICATION

(11) **EP 3 510 960 A1**
(43) Date of publication of application: **17.07.2019**
(21) Application number: 18151374.8
(22) Date of filing: 12.01.2018
(51) Int. Cl.: A61B 18/20, A61B 18/00

(54) **WRINKLE TREATMENT SYSTEM, AND METHODS ASSOCIATED WITH WRINKLE TREATMENT**

(71) Applicant: Koninklijke Philips N.V., 5656 AE Eindhoven (NL)
(72) Inventor: Martens, Peter, 5656 AE Eindhoven (NL); Niessen, Eduard Matheus Johannes, 5656 AE Eindhoven (NL); Verhagen, Rieko, 5656 AE Eindhoven (NL); Varghese, Babu, 5656 AE Eindhoven (NL)
(74) Representative: de Haan, Poul Erik

(57) **Abstract**

A wrinkle tissue preparation method is for preparing wrinkle tissue for a light-based wrinkle tissue treatment method. The preparation involves increasing the amount of target particles in the wrinkle tissue. In an overall wrinkle treatment method, these target particles are then ablated by a subsequent light-based wrinkle tissue treatment method. The preparation stage improves the effectiveness of the light-based treatment for performing wrinkle reduction.

## Description

### FIELD OF THE INVENTION

This invention relates to wrinkle treatment systems and methods.

### BACKGROUND OF THE INVENTION

Light-based wrinkle treatment systems and methods are well-known for providing photothermal tissue treatment.

By way of example, the applicant has developed a novel minimally invasive laser technology for wrinkle reduction by creating isolated lesions in the dermis using laser induced optical breakdown ("LIOB"). The technology is able to effectively improve the appearance of wrinkles, skin texture, and scars without any pain, side effects, or social downtime.

To generate LIOB in soft tissue such as skin, a tightly-focused sub-picosecond to femtosecond laser is used, which leads to plasma formation, plasma expansion, shock wave generation and formation of a microscopic lesion, at a position of a laser focus, e.g., deep in the dermis.

This is a high power process, and hence lower power solutions would be desired.

To this end, many commercial devices and research prototypes relying on sub-nanosecond or so-called picosecond lasers for skin treatment are reported for skin treatment. Initially introduced for the treatment of tattoos, these devices are now also regularly used for the treatment of uneven pigmentation, improvement of acne scars, and treatment of wrinkles.

In these devices, the mechanism involved is for example based on thermomechanical effects resulting in tissue ablation, rather than LIOB.

Such laser induced thermal (as opposed to optical) breakdown or thermomechanical disruption needs an absorbing chromophore such as melanin and the lesions are confined to the chromophores. Since melanin is produced in the basal layer of the epidermis by specialized cells called melanocytes, the maximum treatment depth is confined to the epidermal-dermal junction.

The amount of melanin and its distribution is not uniform throughout epidermis and it can vary for different skin types. The probability of having a target melanin and a corresponding lesion creation in these techniques are lower and thereby limits the treatment efficacy.

Professional skin treatment devices such as Picosure (TM) of the company Cynosure (TM) use weakly focused Gaussian beams for lesion creation at melanin as the target chromophore. Lesions are created only in focus and the probability of finding melanosome in the focal volume is reduced in particular for lightly pigmented skin types.

These thermal based systems still rely on aggressive thermal treatment which can result in damage to tissue beyond the tissue being treated due to thermal diffusion.

Lower energy laser treatment systems have been proposed, in particular which aim to reduce the radiant exposure (also known as fluence), which is defined as the radiant energy delivered to the skin surface per unit area, as well as reducing the exposure time.

For example, WO 2014/160331 A1 discloses systems and methods for treating tissue by concentrating a laser emission to at least one depth at a fluence sufficient to exceed the electron ionization threshold of a target and to create an ablation volume in at least a portion of the target tissue, and controlling the pulse width within the picosecond regime to provide a desired mechanical pressure in the form of shock waves and/or pressure waves which are emitted from the ablation volume to tissue adjacent the target.

In this particular proposed system, the fluence ranges from about 0.8 J/cm² to about 50 J/cm² and the laser emits a pulse width within the range of about 260 ps to about 900 ps.

The pulse width is for example selected to control the magnitude of the pressure wave emission. For example, the pulse width can be selected to maximize a pressure wave emission from the ablation volume to tissue adjacent the target. The picosecond laser pulses have durations below the acoustic transit time of a sound wave through targeted tissues and are capable of generating both photothermal and photomechanical (e.g., shock wave and/or pressure wave) effects through pressures built up in the target.

The main proposed use of the system of WO 2014/160331 A1 is for tattoo removal, but other applications are mentioned such as dermal rejuvenation, as well as other therapeutic applications where an increase in vascularization is desirable.

In a tattoo removal process, the aim is to break the large pigment particles, which are too large to be destroyed by white blood cells, and hence remain under the skin surface to create the permanent tattoo. The tattoo ink particles typically have sizes in the range 100 nm to 10 µm. In this case, the tattoo particles act as absorbing chromophores to result in subsequent opto-thermo-mechanical effects or to create a thermal initiation pathway for optical breakdown.

Treatment of this type has been found to be relatively effective for tattoo removal, but less effective for wrinkle reduction as the lesion creation requires target chromophores such as melanin or blood in the focal volume for thermal initiation.

The amount of melanin and its distribution is not uniform throughout the epidermis and it can vary for different skin types. The probability of having a target melanin and a corresponding lesion creation in these techniques are lower, thereby limiting the treatment efficacy for lightly pigmented skin types.

It would be desirable for a similar system to be made effective for wrinkle treatment (i.e. reduction) applications in particular for lightly pigmented skin types with lower amounts of melanin present in the skin.

### SUMMARY OF THE INVENTION

The invention is defined by the claims.

According to examples in accordance with an aspect of the invention, there is provided a wrinkle tissue preparation method for preparing wrinkle tissue for a light based wrinkle tissue treatment method which performs ablation of target particles beneath the surface of the skin, wherein the method comprises the step of:
increasing the amount of target particles in the wrinkle tissue.

This method provides the introduction of target particles into wrinkle tissue. This has been found to make a subsequent light-based treatment more effective, in particular a treatment which performs particle ablation in order to generate a localized shock wave within the skin tissue.

The target particles for example have a size in the range 30 nm to 10 µm, for example 30 nm to 1 µm, for example 30 nm to 300 nm. Such particles may for example correspond to a thermal relaxation time of less than 10 ns, so that a nanosecond duration laser pulse is suitable. Particles of this size may thus be ablated by a low fluence laser pulse system.

For example, the target particles are ablatable by a laser pulse having a pulse width ranging from 0.2 ps to 100 ns, with examples in the range 260 ps to 2000 ps, for example 260 ps to 900 ps, and a fluence ranging from 0.1 J/cm² to 200 J/cm², for example 0.8 J/cm² to 50 J/cm². The fluence may be below 200 J/cm², e.g. below 100 J/cm², e.g. below 50 J/cm².

In one set of examples, increasing the amount of target particles for example comprises introducing target particles from the outside of the skin associated with the wrinkle tissue into the wrinkle tissue.

This may for example involve injecting or tattooing ink particles. Thus, the preparation may comprise tattooing an area to be subsequently laser treated.

The target particles for example comprise ink having a color black, blue, purple, green and/or red. These colors (of existing tattoo inks) are found to have sizes which are particularly effective for the subsequent ablation process.

The injecting or tattooing may be to a depth beneath the skin surface such that the target particles do not change the visual appearance of the wrinkle. This depth is for example greater than 1 mm (which is typically the maximum depth of a tattoo), and less than 5 mm to have a beneficial surface effect.

The target particles may instead have a color which resembles the natural skin color or wrinkle color. Different sorts of target particles may be mixed to reach a target particle mixture having a color which resembles the natural skin color or wrinkle color. In this way, the ink particles have the minimum visible impact after the wrinkle treatment. However, the wrinkle treatment (being based on a tattoo removal process) may remove the tattoo ink as part of the process.

Instead of a conventional permanent tattoo, another example is to provide a so-called semi-permanent tattoo. This may for example comprise polymethyl methacrylate (PMMA) beads which are known to be nontoxic in human tissue. They may be nano-sized and house pigments.

Other potential candidates are safe, biocompatible and non-toxic pigments such as black and brown pigments using carbon and iron oxide, and yellow and orange pigments using beta-carotene. These are applied to skin via microencapsulation, or by designing tiny beads to transport drugs into the body and release their contents in specific places and at specific times which would keep them from fading into the skin.

In another set of examples, increasing the amount of target particles may comprise activating target particle generation in the wrinkle tissue. This provides an approach which avoids the need to inject into the skin. The natural skin pigment particles are generated by this process.

This process may for example comprise applying UV light to result in tanning of the skin. Alternatively, it may comprise creating petechiae (localized blood spots) by laser or by microneedles. These function to absorb radiation and then create further petechiae.

Activating target particle generation in the wrinkle tissue may comprise:
providing activation crème on the outside of the skin associated with the wrinkle tissue; or
introducing activation particles from the outside of the skin associated with the wrinkle tissue into the wrinkle tissue.

The invention also provides a wrinkle reduction method, comprising:
performing a wrinkle tissue preparation method as defined above; and
performing a light-based wrinkle tissue treatment method.

This method provides a full wrinkle treatment, with the preparation of the skin in advance of the light-based wrinkle treatment.

The light-based wrinkle tissue treatment method for example comprises providing laser pulses to the skin having a pulse width ranging from 0.2 ps to 100 ns and a fluence ranging from 0.8 J/cm² to 50 J/cm².

The method may comprise concentrating the laser emission to target at least one depth in the tissue at a fluence selected to exceed the electron ionization threshold of the target to result in an ablation volume of at least a portion of the target; and
controlling the pulse width to provide a pressure wave emission from the ablation volume to tissue adjacent the target.

The invention also provides a wrinkle treatment system, comprising:
a first apparatus for increasing the amount of target particles in the wrinkle tissue; and
a second apparatus for performing a light-based wrinkle tissue treatment by ablating the target particles.

The first apparatus for example comprises:
an injection system; or
a tattoo system; or
a target particle activation crème.

The second apparatus for example comprises a laser ablation system having a pulse width ranging from 0.2 ps to 100 ns and a fluence ranging from 0.8 J/cm² to 50 J/cm².

These and other aspects of the invention will be apparent from and elucidated with reference to the embodiment(s) described hereinafter.

### BRIEF DESCRIPTION OF THE DRAWINGS

Examples of the invention will now be described in detail with reference to the accompanying drawings, in which:
Fig. 1 shows a wrinkle treatment apparatus; and
Fig. 2 shows a wrinkle treatment method.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a wrinkle tissue preparation method for preparing wrinkle tissue for a light based wrinkle tissue treatment method. The preparation involves increasing the amount of target particles in the wrinkle tissue. In an overall wrinkle treatment method, these target particles are then ablated by a subsequent light-based wrinkle tissue treatment method. The preparation stage improves the effectiveness of the light-based treatment for performing wrinkle reduction.

The invention is based on the recognition that that wrinkles naturally contain a relatively low amount of pigment. This fact, in combination with the fact that the effectiveness of the treatment described in WO 2014/160331 A1 is proportional to the amount of pigment or tattoo particles present in the treated area, explains why the treatment described in WO 2014/160331 A1 is less effective for treating wrinkles than for tattoo removal.

The invention is based on actively increasing the number of target particles in wrinkles so that the effectiveness of a laser-based skin treatment method, in particular a picosecond laser pulse ablation system, can be improved for wrinkles.

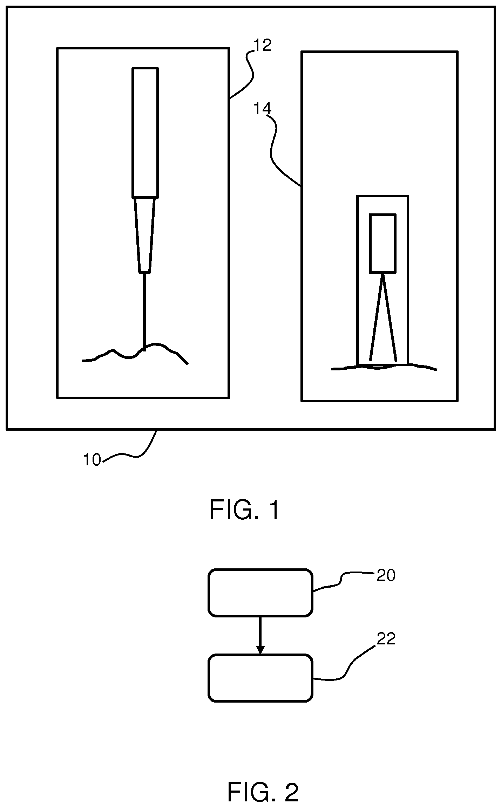

Fig. 1 shows a wrinkle treatment system 10, comprising a first apparatus 12 for increasing the amount of target particles in the wrinkle tissue and a second apparatus 14 for performing a light-based wrinkle tissue treatment by ablating the target particles.

The second apparatus 14 may comprises a laser ablation system having a pulse width ranging from 0.2 ps to 100 ns and a fluence ranging from 0.8 J/cm² to 50 J/cm². This may be of the type disclosed in WO 2014/160331 A1.

Actively increasing the number of target particles in wrinkles can be achieved in several ways. In particular, the first apparatus 12 may comprise a controlled release system, an injection system, a tattoo system, a natural pigment activation system, a natural other particle activation system, or a target particle activation crème.

For a controlled release system, providing particles or colorants to the skin via the skin surface may make use of controlled release mechanisms or transdermal drug delivery methods.

For an injection or tattoo system, the particles are for example ink pigment particles. These ink pigment particles are selected to be sensitive to the laser treatment.

One example of activation particles is to provide a so-called semi-permanent tattoo. This may for example comprise polymethyl methacrylate (PMMA) beads which are known to be nontoxic in human tissue. They may be nano-sized and house pigments.

For an activation crème, the skin is activated to produce more natural pigment particles in the wrinkles. This activation may be from the outside of the skin using a crème which activates pigment generation, or from the inside of the skin by introducing particles in the wrinkle via the skin which particles activate pigment generation.

Absorbing compounds may be provided to the skin either directly at the location or via the bloodstream.

The physical approach for delivering a target (whether particles or an activation crème) can be via abrasion, microscissuining, electroporation, iontophoresis, pressure waves, sonophoresis, or micro needling. By wat of example, reference is made to Journal of Controlled Release Volume 242, 28 November 2016, Pages 3-15; "Nanocarriers for drug delivery into and through the skin - Do existing technologies match clinical challenges?"

For a natural pigment activation system, the process may for example comprise applying UV light to result in tanning of the skin.

For a natural particle activation system, the process may comprise providing laser induced thermal breakdown to create petechiae (localized blood spots). These function to absorb radiation and then create further petechiae.

For an injection or tattooing approach, the tattooed ink particles may have a color which is close to or resembles the natural skin/wrinkle color. This has as advantage that the presence of the tattooed particles for improving the wrinkle treatment is less notable. However, because of this color resemblance between the skin and tattooed particles, it may be difficult to judge for the tattoo practitioner whether a position/area has been treated or not.

If the tattoo particles visibly behave differently to the skin under certain lighting conditions (e.g. UV), the lighting conditions may be adapted to enable distinction between treated and untreated regions.

If the tattoo ink particles do not visibly behave differently to the skin under any possible lighting conditions, then other particles may be added to the tattoo particles which other particles behave differently to the skin under abnormal lighting conditions such as when exposed to UV light. These particles contains fluorescent particles which emit longer wavelength radiation (visible light) after absorbing short wavelength radiation such as in the in the ultraviolet region of the spectrum.

It is beneficial that these additional particles remain present in the skin only for a long enough period of time to allow subsequent wrinkle treatment, but short enough to not be visible after the total treatment. Thus, it is beneficial that these additional particles are themselves quickly removed from the skin by natural processes. However, if this is not the case, these additional particles may be removed by the subsequent wrinkle treatment, in the same way that the wrinkle treatment functions as a tattoo removal process.

Different ink colors respond differently to a tattoo removal treatment.

For example, it has been reported than black, blue, purple, green and red inks are the easiest to remove, while yellow and white are the hardest. The added target particles may thus be selected from the easily removable inks as listed above. The presence of these inks make the subsequent laser treatment process more effective, and it means the inks are themselves removed as part of the wrinkle treatment treatment. Black is for example easy to remove because it absorbs all radiation whereas white is most difficult.

The mean diameter of the target particles reduces after successive laser treatments. The maximum diameter of a granule that can be absorbed by the lymphatic system is approximately 0.4 µm, so that any laser procedure that tries to reduce the size further is of little clinical use.

Another option is to inject the ink particles to a depth under the skin into the wrinkle such that the ink does not change the visual appearance of the wrinkle, but the ink can still act as target particle in the light-based treatment. For example, the particles may be provided at a depth of more than 2 mm below the skin surface, for example in the range 2 mm to 5 mm.

Another option is to provide an ink color which matches the skin color. This may be achieved by mixing different sorts of target particles to reach a target particle mixture having a color which resembles the natural skin color or wrinkle color.

The light-based system of WO 2014/160331 is discussed above. Reference is also made to the related patents US 7 586 957 and US 7 929 579, which relate to the details of the laser apparatus.

There are other similar commercially available picosecond pulse low radiance skin treatment systems. These are available from Cynosure (TM) called the "PicoSure" (TM) system, Syneron Candela (TM) called the "PicoWay" (TM) system and Cutera, Inc. (TM) called the "PicoGenesis" (TM) system.

Examples of the laser pulse parameters for some of the commercially available systems are:

| System | Pulse duration (ps) | Pulse energy (mJ) | Wavelength (nm) | Fluence (J/cm²) |
|---|---|---|---|---|
| PicoSure | 550-750 | 165-200 | 532, 755, 1064 | 0.8-50 |
| PicoWay | 300-500 | 50-400 | 532, 1064 | 0.2-100 |
| PicoGenesis | 750-2000 | 300-600 | 532, 670, 1064 | 1.2-150 |

The duration is for example in the range 0.2ps to 100ns, with typical existing laser systems operating in the range 300 ps to 2000 ps (2ns) as shown in the table above. A laser pulse duration in the ns range is for example preferred as this corresponds to an energy delivery suitable for particle destruction at the typical sizes of the target particles. This makes Q-Switched lasers particularly suitable.

Different wavelengths may be used for different treatments in these known systems, in particular for different skin types, for example as defined by the Fitzpatrick scale I to VI where type I is the most sensitive skin and type VI is the least sensitive skin. The shorter wavelengths are typically used for more sensitive skin types e.g. I to III. The wavelength may also be selected depending on the tattoo pigment to be removed.

Thus, the operating conditions of the laser based system may also be selected in the approach of the invention having regard to the target particles being used and the skin type being treated.

The pulse width for example ranges from 0.2 ps to 100 ns, e.g. 1 ps to 10 ns, e.g. 260 ps to 2000 ps, e.g. 300 ps to 775 ps, e.g. 450 ps to 600 ps, e.g. 260 ps to 500 ps. The fluence for example ranges from 0.1 J/cm² to 200 J/cm², e.g. 0.2 J/cm² to 150 J/cm², e.g. 0.8 J/cm² to 50 J/cm², e.g. 0.8 J/cm² to 25 J/cm², e.g. 0.8 J/cm² to 10 J/cm².

Fig. 2 shows a wrinkle reduction method, comprising:
in step 20, performing a wrinkle tissue preparation method as described above; and
in step 22 performing a light-based wrinkle tissue treatment method as also discussed above.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope.

## Claims

1. A wrinkle tissue preparation method for preparing wrinkle tissue for a light based wrinkle tissue treatment method which performs ablation of target particles beneath the surface of the skin, wherein the method comprises the step of:
increasing the amount of target particles in the wrinkle tissue.

2. A method as claimed in claim 1, wherein the target particles have a size in the range 30 nm to 10 µm.

3. A method as claimed in claim 1 or 2, wherein the target particles are ablatable by a laser pulse having a pulse width ranging from 0.2ps to 100ns and a fluence ranging from 0.1 J/cm² to 200 J/cm².

4. A method as claimed in any preceding claim, wherein increasing the amount of target particles comprises introducing target particles from the outside of the skin associated with the wrinkle tissue into the wrinkle tissue.

5. A method as claimed in any preceding claim, wherein the target particles comprise ink particles, and the method comprises injecting or tattooing.

6. A method as claimed in claim 5, wherein:
the target particles comprise ink having a color black, blue, purple, green and/or red; and/or
the injecting or tattooing the ink particles is to a depth beneath under the skin such that the target particles do not change the visual appearance of the wrinkle.

7. A method as claimed in claim 4, wherein the target particles have a color which resembles the natural skin color or wrinkle color.

8. A method as claimed in claim 1 or 2, wherein increasing the amount of target particles comprises activating target particle generation in the wrinkle tissue.

9. A method as claimed in claim 8, wherein activating target particle generation in the wrinkle tissue comprises:
providing activation crème on the outside of the skin associated with the wrinkle tissue; or
introducing activation particles from the outside of the skin associated with the wrinkle tissue into the wrinkle tissue.

10. A wrinkle reduction method, comprising:
(20) performing a wrinkle tissue preparation method as claimed in any preceding claim; and
(22) performing a light-based wrinkle tissue treatment method.

11. A method as claimed in claim 10, wherein the light-based wrinkle tissue treatment method comprises providing laser pulses to the skin having a pulse width ranging from 0.2ps to 100ns and a fluence ranging from 0.1 J/cm² to 200 J/cm².

12. A method as claimed in claim 11, comprising:
concentrating the laser emission to target at least one depth in the tissue at a fluence selected to exceed the electron ionization threshold of the target to result in an ablation volume of at least a portion of the target; and
controlling the pulse width to provide a pressure wave emission from the ablation volume to tissue adjacent the target.

13. A wrinkle treatment system (10), comprising:
a first apparatus (12) for increasing the amount of target particles in the wrinkle tissue; and
a second apparatus (14) for performing a light-based wrinkle tissue treatment by ablating the target particles.

14. A system as claimed in claim 13, wherein the first apparatus comprises:
an injection system; or
a tattoo system; or
a target particle activation crème.

15. A system as claimed in claim 13 or 14, wherein the second apparatus comprises a laser ablation system having a pulse width ranging from 0.2ps to 100ns and a fluence ranging from 0.1 J/cm² to 200 J/cm².
